(19) Europäisches Patentamt
European Patent Office
Office européen des brevets

(11) EP 3 827 748 A1

(12) EUROPEAN PATENT APPLICATION

(43) Date of publication:
02.06.2021 Bulletin 2021/22

(51) Int Cl.:
A61B 5/107 (2006.01)       A61B 5/00 (2006.01)
A61J 9/00 (2006.01)        G16H 20/13 (2018.01)

(21) Application number: 19211379.3

(22) Date of filing: 26.11.2019

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
KH MA MD TN

(71) Applicant: Koninklijke Philips N.V.
5656 AG Eindhoven (NL)

(72) Inventors:
• TIEMANN, Christian Andreas
  5656 AE Eindhoven (NL)
• WOUTERS, Cornelis Bernardus Aloysius
  5656 AE Eindhoven (NL)

(74) Representative: Philips Intellectual Property &
Standards
High Tech Campus 5
5656 AE Eindhoven (NL)

(54) **INFANT MONITORING SYSTEM DURING FEEDING**

(57)    A monitoring system is provided for monitoring an infant during bottle feeding. Based on bottle orientation information and movement information in respect of the feeding bottle during feeding, infant orientation information in respect of the infant is obtained.

FIG. 1

## Description

FIELD OF THE INVENTION

This invention relates to feeding bottles and in particular relates to a system for monitoring the orientation of an infant when drinking from a bottle.

BACKGROUND OF THE INVENTION

It is desirable when bottle feeding an infant to know how well the infant is drinking. It is known to monitor drinking performance and to provide feedback to the parent. One known example is in the form of a sleeve for the infant bottle, which incorporates a load cell, for measuring the weight of the milk contained by the bottle before and after feeding, and thereby to calculate the milk volume consumed by the infant. The sleeve also contains an accelerometer, to give feedback to the parents in respect of the correct bottle angle, as well as for monitoring the drinking behavior of the child by looking at the bottle movements (e.g., to identify drinking bursts and pauses). The system also allows data to be sent to a companion app for analysis and visualization.

The quality of bottle feeding is influenced by the position or orientation of the infant while drinking. It is for instance advised to position the infant relatively upright. This will prevent that milk flows into the inner ears, where it may cause an infection. The position of the infant may also influence the chance of excessive air intake, chance of reflux, and risk of choking.

It would therefore be interesting to automatically determine the position of the infant during feeding. This information could be used to give feedback during a feed as to whether the position is appropriate or not, and to advise repositioning if appropriate (for example if the infant is restless). Tracking the infant orientation info over time (optionally in combination with other feeding cues) can help parents to understand what is most comfortable for their child. A camera system could be used for the orientation detection, but this is not a convenient and desired solution.

One parameter of interest is the tilt angle of the infant about a vertical axis. This tilting may occur when the infant is leaning against the chest of the parent while drinking. However, there are several challenges to estimate the infant tilt angle from an accelerometer coupled to the bottle, in particular because of the rotational freedoms in the system.

Another parameter of interest is the angle of the infant to the horizontal, i.e. how upright an infant is sitting during a feed. If the longitudinal axis of the bottle is perpendicular to the longitudinal body axis of the infant (i.e. approximately a straight representation of the spine), the bottle angle is then identical to the inclination angle. In practice, however, these axes will not be perfectly perpendicular to each other.

It would also be desirable to be able to monitor accurately one or both of these body orientation parameters in real time during a feed.

SUMMARY OF THE INVENTION

The invention is defined by the claims.

According to examples in accordance with an aspect of the invention, there is provided a monitoring system for determining infant orientation information during bottle feeding, comprising:

a sensor arrangement for obtaining bottle orientation information and movement information in respect of the feeding bottle during feeding; and
a processor adapted to identify from the sensor arrangement sensor signals infant orientation information in respect of the infant; and
an output interface for providing output information dependent on the infant orientation information.

The invention is based on the recognition that although the orientation of an infant is not directly linked to (and hence easily derivable from) the orientation of the bottle from which they are feeding, the infant orientation can be derived from the combination of orientation information and movement information relating to the bottle. In particular, the process of drinking from a bottle induces movements in the bottle in directions which depend on the orientation of the infant and the orientation of the infant relative to the bottle. Thus, the orientation information and movement information relating to the bottle can be used to derive information about the infant.

The sensor arrangement for example comprises a three-axis motion sensor such as a three-axis accelerometer and/or a three-axis gyroscope.

The output interface may comprise a wireless transmitter for sending the output information to a remote device for presentation to a user. This information is for example objective feedback on infant position and may be used to help a parent understand what is most comfortable for their child.

In a first example, the processor may be adapted to identify infant orientation information comprising an angle of tilt of a body axis of the infant about a vertical axis. This is a left-right tilt of the infant. The body axis is a general axial representation of the body of the infant, for example aligned with the end-to-end direction of the spine.

For this purpose, the processor may be adapted to translate the sensor arrangement signals to a reference coordinate system to compensate for rotation about a bottle longitudinal axis during a feed. If two-axis accelerations are being monitored in a plane of the base of the bottle, it is not initially known how the bottle is held. In particular, the rotational position about the bottle longitudinal axis is not known. By translating to a reference coordinate system, this ambiguity is resolved.

The processor may then be adapted to identify components of movement corresponding to longitudinal movement parallel to a body axis of the infant caused by jaw movement and thereby to determine the orientation of the body axis of the infant (relative to the known reference coordinate system). These longitudinal movements can be detected and then interpreted using the reference coordinate system.

The processor is for example adapted to identify the components of movement by finding a minimum correlation between components of movement in orthogonal directions in a plane perpendicular to a longitudinal axis of the bottle. By finding an orientation with lowest correlation, the direction in which these components of movement are principally oriented is then found (within the reference coordinate system). This then identifies the orientation of the infant in the reference coordinate system.

In another example, the processor is adapted to identify infant orientation information comprising an angle of tilt of a body axis of the infant about a horizontal axis. This is a forward-back tilt of the infant. This is typically close to orthogonal the body axis of the infant. However, for greater accuracy, the processor is preferably adapted to identify an offset between a longitudinal axis of the bottle and an axis perpendicular to the body axis of the infant.

The processor may be adapted to identify the offset by using a regression model which models the way bottle movements vary during feeding in dependence on the offset angle.

The monitoring system is for example arranged as a sleeve for mounting around a feeding bottle.

The invention also provides a method for determining infant orientation information during bottle feeding, comprising:

obtaining bottle orientation information and movement information in respect of the feeding bottle during feeding;
identifying from the bottle orientation information and movement information infant orientation information in respect of the infant; and
providing output information dependent on the infant orientation information.

The method may comprise:

identifying infant orientation information comprising an angle of tilt of a body axis of the infant about a vertical axis; and/or
identifying infant orientation information comprising an angle of tilt of a body axis of the infant about a horizontal axis.

The invention also provides a computer program comprising computer program code means which is adapted, when said program is run on a computer, to implement the method defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a feeding bottle mounted in a sleeve which functions as a monitoring system;
Figure 2 shows one possible set of signals from a combination of a 3-axis acceleration sensor and a 3-axis gyroscope;
Figure 3 shows a front view of an infant in an upright position (left image) and in a tilted position (right image);
Figure 4 shows a bottom of the bottle, and represents the x and y axes, which are in the plane perpendicular to the longitudinal axis of the bottle;
Figure 5 is used to explain a coordinate system translation;
Figure 6 shows an example of a real feed of accelerometer signals;
Figure 7 shows how drinking results in repetitive bottle motions;
Figure 8 shows a plot of correlation versus matrix rotation from a real feed;
Figure 9 shows a scatter plot of estimated tilt versus actual imposed tilt;
Figure 10 represents an inclination angle $\alpha$ and a corresponding bottle angle $\beta$;
Figure 11 shows how the bottle angle can be determined using the gravity vector;
Figure 12 shows an example where the bottle is placed slightly more upright (by angle $\delta$) with respect to the infant's inclination;
Figure 13 shows the results of a test to show the operation of the determination of the inclination angle;
Figure 14 shows the processed acceleration and gyroscope signals in which gravity effects have been filtered out;
Figure 15 shows the accelerometer and gyroscope signal contributions in a statistical form; and
Figure 16 shows the results of determining the inclination angle for the test data.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a monitoring system for monitoring an infant during bottle feeding. Based on bottle orientation information and movement information in respect of the feeding bottle during feeding, orientation information in respect of the infant is obtained (named "infant orientation information" in this document).

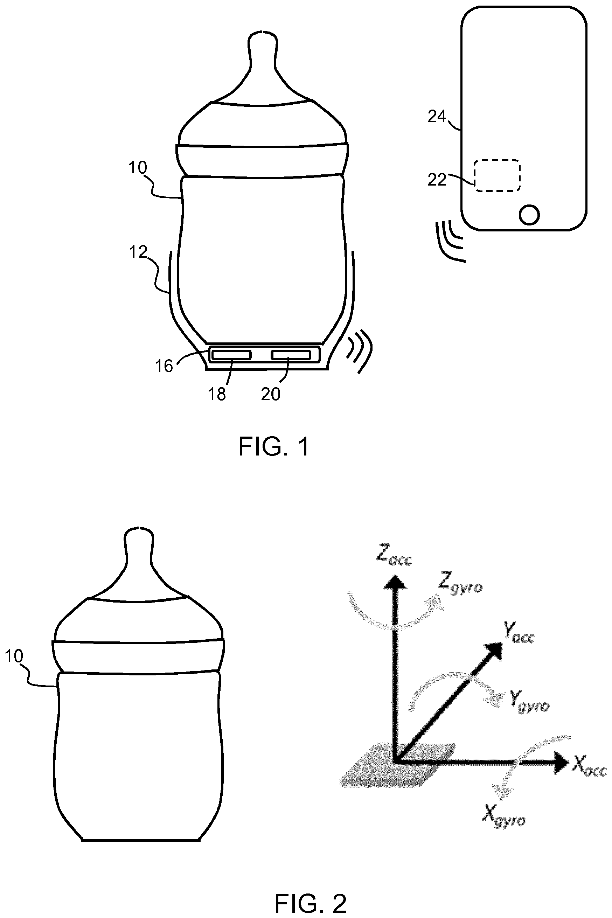

Figure 1 shows a feeding bottle 10 mounted in a sleeve 12 which functions as a monitoring system. The sleeve 12 surrounds the feeding bottle 10.

A monitoring unit 16 is in this example provided in the base of the sleeve 12, and comprises a sensing arrangement 18 for sensing bottle orientation and movement (of a bottle coupled to the monitoring unit), and an output interface 20. The monitoring unit 16 may be incorporated anywhere in or on the sleeve.

The base part of the sleeve for example also includes a battery, and optionally means for providing visual feedback to the user via LEDs. The output interface 20 may comprise this LED arrangement. However, a preferred implementation instead (or additionally) has an output interface which communicates the results wirelessly to a smartphone 24 or tablet as shown.

A processor 22 determines infant orientation relating to the infant and then provides information to the user. This information may be the infant orientation itself, or it may be advice or information which can be derived from knowledge of the infant orientation.

In the example shown, the processor 22 is the processor of a mobile phone 24 which communicates wirelessly with the monitoring unit 16. Thus, the sleeve locally detects motion, and the remote processor analyzes the motion to derive the infant orientation. Thus, a parent feeding an infant may monitor on their mobile phone information relating to the feeding, in particular the infant orientation. This is of course only one example. The processor 22 which analyses the motion data could also be located on the sleeve and integrated with the monitoring unit 16. In this case only the infant orientation information needs to be transmitted to the mobile phone. In this case, raw motion data does not have to be transmitted to the phone, saving time and battery life.

The sensing arrangement 18 preferably comprises a 3-axis acceleration sensor and/or a 3-axis gyroscope.

Figure 2 shows one possible set of signals from a combination of a 3-axis acceleration sensor and a 3-axis gyroscope. An image of the bottle 10 is shown to illustrate the 3-axis orientations.

This arrangement gives three linear acceleration signals $X_{acc}$, $Y_{acc}$, $Z_{acc}$ and three angular velocity signals $X_{gyro}$, $Y_{gyro}$ and $Z_{gyro}$. The sensor arrangement is generally an inertia measurement unit and/or force or acceleration measurement unit.

Different implementations of the system may make use of acceleration sensors only, or gyroscope sensors only, or both.

The processor 22 is programmed generally to determine infant orientation information in respect of the infant during a feed.

Two types of infant orientation information are explained below. A first type of infant orientation information is the tilt angle about a vertical axis.

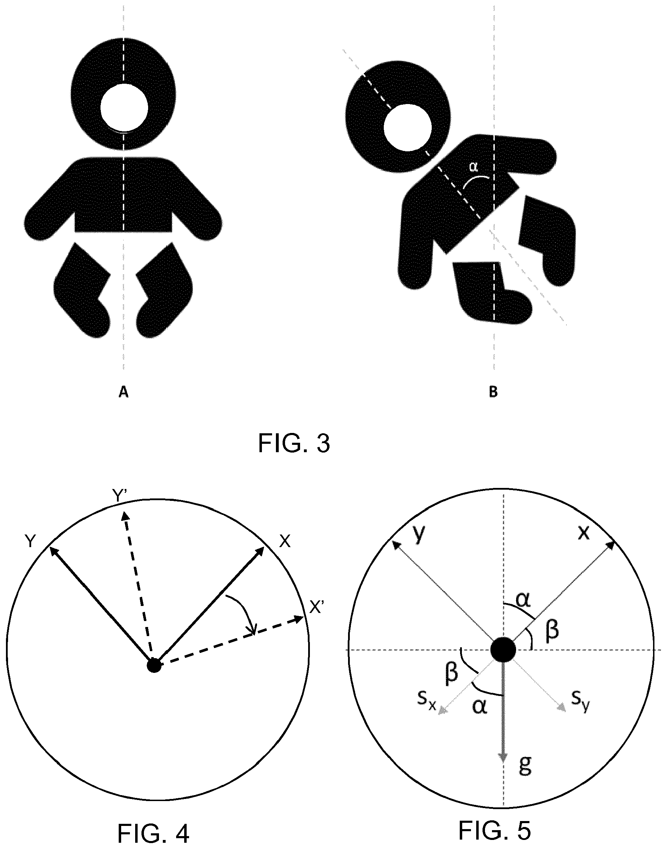

Figure 3 shows a front view of an infant in an upright position (left image) and in a tilted position (right image) with a tilt of angle $\alpha$ to the vertical. Tilting may occur when the infant is leaning against the chest of the parent while drinking.

By determining this tilt angle, information and guidance on infant positioning can be given to the mother, and may also be used to enhance the detailed analysis of infant drinking behavior over time.

There are several challenges to estimate this infant tilt angle using accelerometers due to several rotational freedoms in the system. First, there is not a unique mapping from a bottle position to an infant tilt angle. For instance, when looking at Figure 3, both infants have a different tilt angle while the bottle is in the same position.

Second, the directions of the axes of the accelerometer are not uniquely defined. The sleeve 12 is a round object which is more or less rotation-symmetric, and therefore it can freely rotate around the bottle (hence the x-axis and y-axis can freely rotate around the z-axis). Every feed, the parent may attach the sleeve differently to the bottle. Moreover, even if there was only one way to connect the sleeve to the bottle, the bottle-sleeve combination is more or less rotation-symmetric as well. During every feed, the parent may hold the bottle differently, and also during the feed the bottle may rotate around its longitudinal axis (z-axis). This has been regularly observed in data acquired during home studies by the applicant.

Note that this applies not only to a sleeve, but also to any mounting of a sensor arrangement; the bottle can always be held in different rotational positions.

The invention is based on the recognition that movement information may be used to enable infant orientation to be determined, including this tilt angle.

Figure 4 shows a bottom of the bottle, and represents the x and y axis, which are in the plane perpendicular to the longitudinal axis of the bottle (e.g. the base of the bottle). As shown in Figure 4, the x-axis and y-axis can rotate freely around the longitudinal axis of the bottle from x,y to x',y'. The bottle orientation can change between feeds, but also during feeds.

A first part of the processing is to translate to a fixed orientation system, in which the translated y value is aligned vertically and the translated x value is aligned horizontally. In this way, the sensor arrangement signals are translated to a reference coordinate system to compensate for rotation about the bottle longitudinal axis during a feed.

Figure 5 is used to explain this coordinate system translation. In this example the x and y axes are counter-clockwise rotated by angle $\beta$ with respect to the desired horizontal and vertical axis. The gravitational acceleration results in signals $S_x$ and $S_y$ in the x and y directions, respectively. The angle between x and the desired reference coordinate x-axis needs to be determined (angle $\beta$ in Figure 5). This can be done making use of the gravitational acceleration g measured by the accelerometer. The gravity vector is offset by angle $\alpha$ from the $S_x$ signal. Based on trigonometric rules the angle $\beta$ can be determined as follows:

$$\beta = 90 - \alpha$$

$$\alpha = \tan^{-1}\left(\frac{|S_y|}{|S_x|}\right)$$

The components Sx and Sy can be obtained by low pass filtering (since the gravity vector is constant) thereby excluding movements of the infant when determining the coordinate system translation.

Gravity will induce an offset in the accelerometer axes. The aim is thus to extract these offsets (which have a low frequency). The faster movements due to drinking are removed due to the low pass filtering.

Subsequently, the signals in the x and y direction can be rotated such that these reflect horizontal and vertical accelerations because of the mapping to the reference coordinate system. The signals can be rotated making use of the following counter-clockwise rotation matrix:

$$R = \begin{pmatrix} \cos\beta & -\sin\beta \\ \sin\beta & \cos\beta \end{pmatrix}$$

The rotation angle may need to be adjusted depending on the quadrants in which the x and y axis are located.

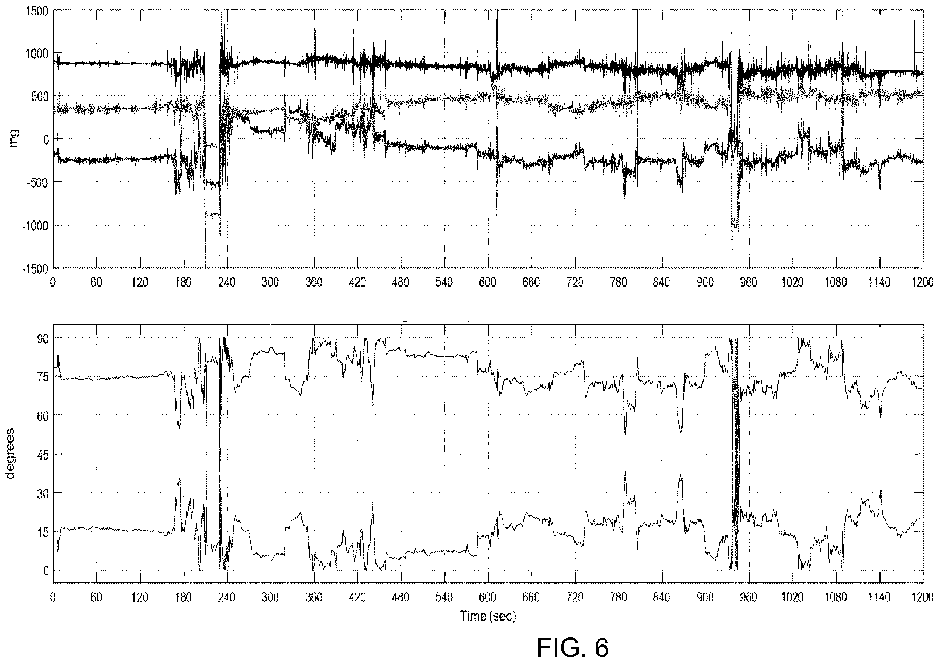

Figure 6 shows an example of a real feed. The top graph shows the three accelerometer signals. The top plot is the x-axis, the middle plot is the z-axis and the bottom plot is the y-axis.

The bottom graph shows the angle of the x and y directions with respect to the horizontal axis during the feed. The top plot is the x-axis angle relative to horizontal, and the bottom plot shows the y-axis angle relative to the horizontal axis.

The bottle orientation angle can vary considerably throughout the feed as can be seen. Therefore, the angle determination and axis rotation may be performed every sample, such that a fixed reference coordinate system is obtained throughout the whole feed.

The infant tilt angle may be derived by making use of knowledge of typical bottle motions induced during feeding. Drinking results in repetitive bottle motions. The sucking behavior involves a front to back movement of the tongue which results in a motion along the longitudinal axis of the bottle (z-axis). Jaw movements also induce up and down movements, typically along the longitudinal axis of the infant.

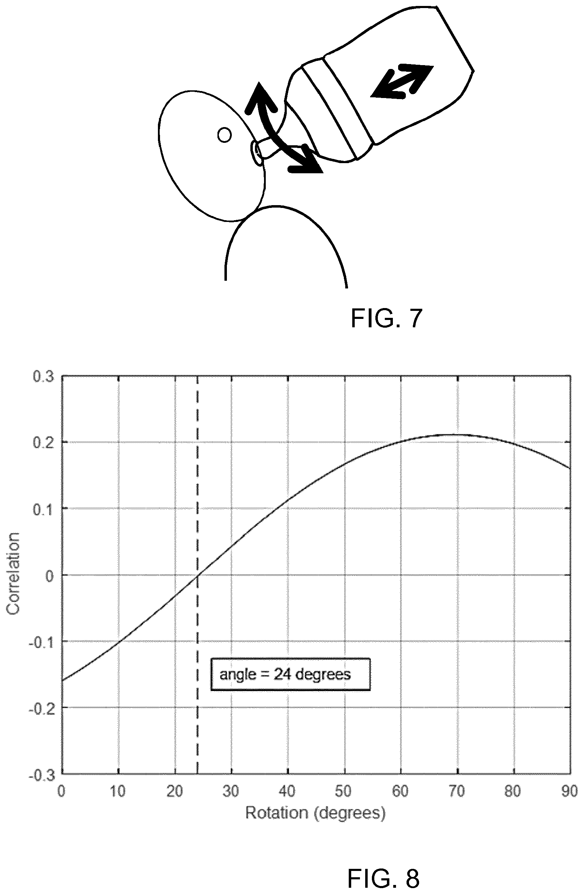

Figure 7 shows how drinking results in these repetitive bottle motions, which are mainly visible in the longitudinal axis of the bottle and the body axis of the infant. The longitudinal movement of the infant is of particular relevance for determining the tilt angle as defined above. The body axis of the infant corresponds to the vertical axis rotated by the tilt angle to be determined.

The way the infant tilt can be derived can be understood by considering different scenarios. If the y-axis is aligned with the longitudinal infant axis, the bottle movement would be clearly visible in that direction, while in the perpendicular direction (x-axis) there would be only limited amount of motion visible (mostly noise). Consequently, there would be no correlation between the x and y signals. If the y-axis is not aligned with the longitudinal infant axis, it would be expected that the bottle movements are to some extend visible in both the x and y directions, and they would bear some correlation.

This principle can be used to find the tilt angle. In particular, components of this movement can be obtained by finding a minimum correlation between components of movement in these orthogonal x and y directions (perpendicular to the longitudinal axis of the bottle). By applying different rotation matrices to the acceleration signals, the rotation angle at which the correlation coefficient becomes minimum (or zero) is obtained, and from this the infant tilt angle can be determined (relative to the now known reference coordinate system).

Figure 8 shows a plot of correlation versus matrix rotation angle from a real feed. The correlation between the time series signals of the x and y accelerometer signals is plotted for different rotations.

The amount of data needed to implement the correlation is related to the sucking frequency which is typically between 1-2Hz. Sufficient variation in the data is needed to detect the correlation. After a small number of infant sucks, the movement along the longitudinal axis of the infant may be captured and it will then be possible to detect the correlation. Thus a few seconds of data while the infant is drinking will be sufficient. During a long break it will of course not be possible to update the tilt angle, because there is no movement information.

The rotation at zero correlation provides an estimation of the infant tilt angle. This graph shows the results of a real feed for which the tilt angle is 24 degrees.

Experiments have been conducted in a laboratory setting to test the tilt estimation method explained above. In these experiments, the bottle sleeve was connected to a holder system which represented the infant. A tilt angle was imposed on the holder system, after which bottle movements were induced to mimic sucking behavior. Subsequently, the algorithm was applied to the measurement data to estimate the imposed tilt angle. The experiment was repeated for different tilt angles (and different bottle angles).

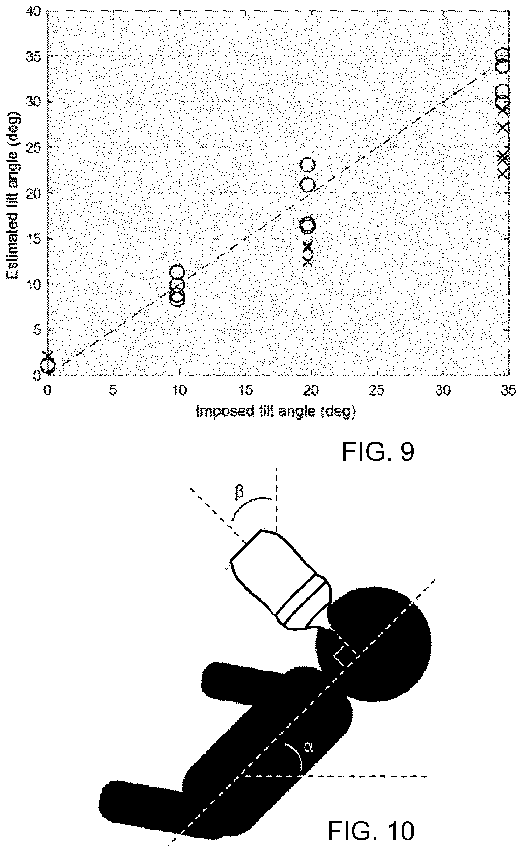

Figure 9 shows a scatter plot of estimated tilt versus actual imposed tilt. The circles correspond to measurements where the head of the infant is in the same plane as the body, i.e., there is no sideway rotation of the head. These estimations are fairly close to the real tilt angles. In case the head is rotated sideways with respect to the body, the head is in a different tilt position, which will induce an error. The crosses correspond to measurements where the head was rotated 45 degrees, resulting in an under estimation of the tilt angle. However, there is still a strong correlation between the imposed and estimated tilt angle.

Thus, in general, the estimated tilt angles match well with the imposed ones. There are two conditions under which less accurate estimations are obtained. First, if the bottle is almost in a vertical position, inaccurate estimations are obtained. This is because the x and y axis experience the same gravity component. However, this would mean that the infant is lying flat, which is an uncommon drinking position. Another source of noise is introduced when the head of the infant is turned sideways with respect to the body as mentioned above, because the bottle is then moving in a different plane compared to the body, which will cause an under- or overestimation of the body tilt angle.

The example above estimates a first type of infant orientation information; the tilt angle of the infant from the vertical. A second type of orientation information of interest is the upright position angle (the inclination to the horizontal) of the infant during bottle feeding.

Figure 10 represents this inclination angle $\alpha$ and a corresponding bottle angle $\beta$. Estimating both the tilt angle and inclination angle for example gives a more complete picture of the position/orientation of the infant during feeding.

It can also be challenging to determine how upright an infant is sitting during a feed. If the longitudinal axis of the bottle is perpendicular to the body axis of the infant, the bottle angle $\beta$ to the vertical is identical to the inclination angle $\alpha$. In practice, however, these axes will not be perfectly perpendicular to each other. There can be some variation in how the bottle is placed in the infant's mouth which will introduce an under or overestimation of the inclination angle.

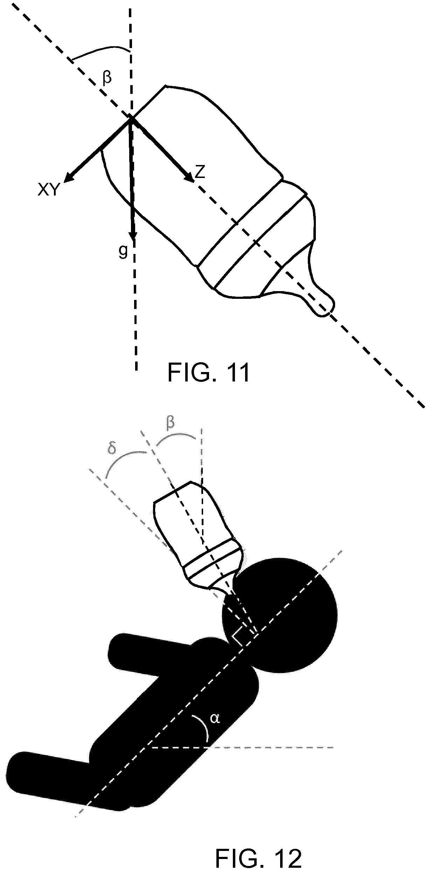

Figure 11 shows how the bottle angle $\beta$ can be determined using the gravity vector. Depending on the bottle angle, the gravity induced accelerations will be distributed differently over the three components of the accelerometer. Based on trigonometric rules the bottle angle can be calculated as follows:

$$\beta = \tan^{-1}\left(\frac{\sqrt{X_{acc}^2 + Y_{acc}^2}}{Z_{acc}}\right)$$

As mentioned above, if the longitudinal axis of the bottle is perpendicular to the body axis of the infant, the bottle angle $\beta$ is identical to the inclination angle $\alpha$. It is noted however that this only holds when the infant is not rotated around its body axis (i.e., not rotated sideward). A sideward rotation of the infant will induce a slight overestimation of the inclination angle (e.g., $\approx$1.5% for a 10 degree rotation).

In practice, there will not be a perfect perpendicular alignment, which will introduce an underestimation or overestimation of the inclination angle.

Figure 12 shows an example where the bottle is placed slightly more upright (by angle $\delta$) with respect to the infant's inclination. Describing the inclination angle as a function of the bottle angle will result in an underestimation in this case. Instead, the inclination angle $\alpha$ is given by the sum of $\beta$ and $\delta$. The challenge is to determine 8 because it cannot be straightforwardly derived from the accelerometer.

As for the tilt angle determination, the angle $\delta$ is estimated, and hence the overall inclination angle is estimated, by analyzing bottle motions during the feed induced by the sucking behavior of the infant. The approach is based on the recognition that the relative magnitude of linear accelerations and angular velocities in the three different directions changes when the bottle is positioned at different angles in the infant's mouth. Hence, features can be derived from the motion data to estimate the angle $\delta$. Modeling techniques like regression can be used to define the relation between the motion features and angle $\delta$.

To demonstrate the viability of the approach, a test was performed with repetitive motions applied to a bottle teat to mimic the sucking behavior of an infant. The test consisted of three phases during which the bottle angle changed, while the inclination angle remained constant.

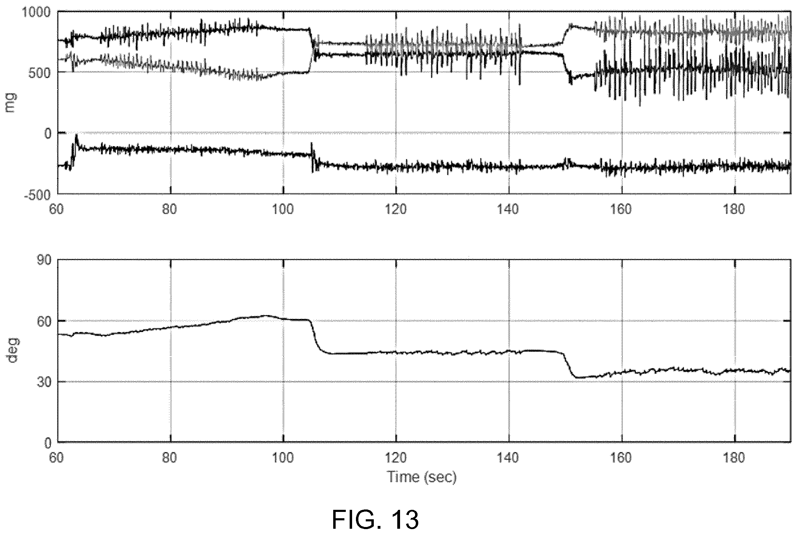

Figure 13 shows the results. The top graph of Figure 13 shows the accelerations for all three axes during the three phases. The top plot is the z-axis, the middle plot is the y-axis and the bottom plot is the x-axis. The bottom graph shows the calculated bottle angle.

Based on the acceleration data the bottle angle was calculated, which is shown in the bottom graph. The first phase ends at around t=105s, and the second phase ends at around t=150s.

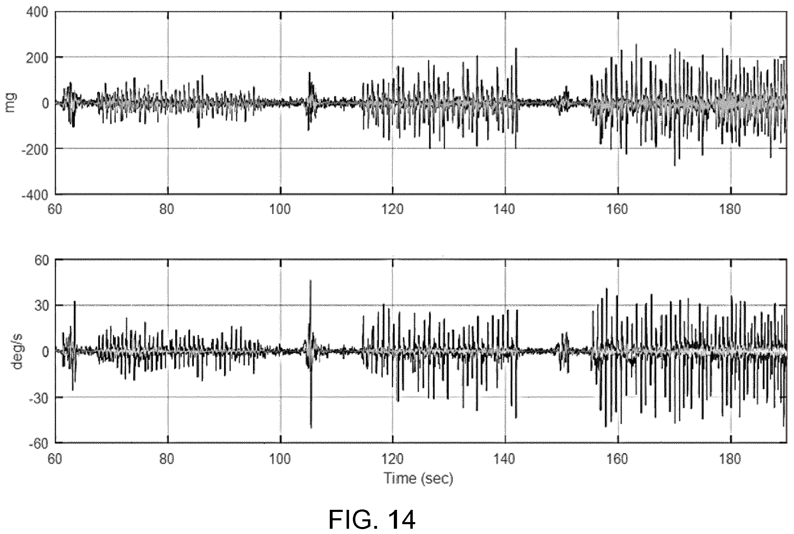

Figures 14 to 16 show how the inclination angle is caclulated. Figure 14 shows processed acceleration and gyroscope signals in which gravity effects have been filtered out. Several motion components change during the different phases. The top graph shows the three-axis acceleration signals. They generally overlap. The bottom graph shows the three axis gyroscope signals, which again generally overlap.

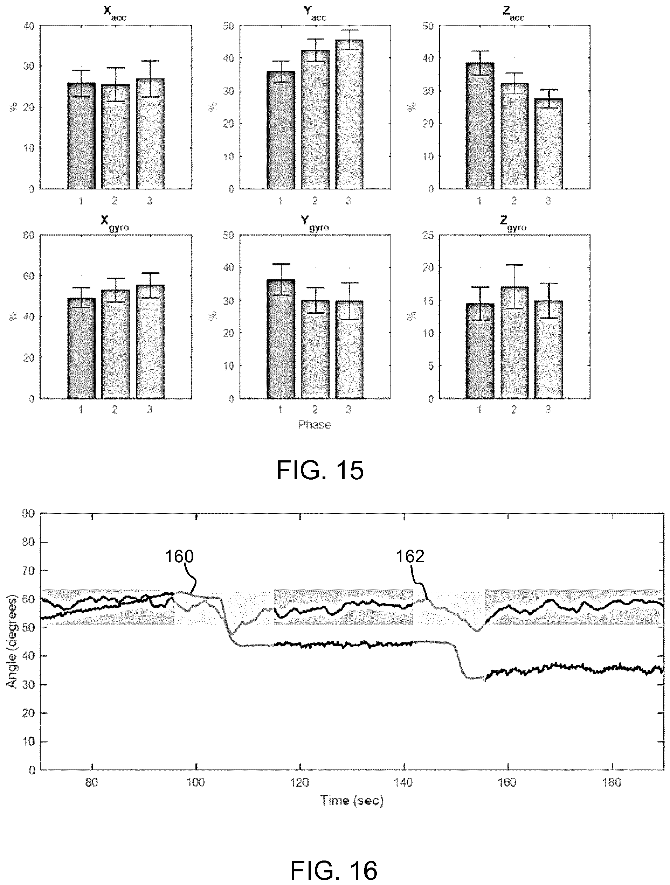

Figure 15 shows an analysis of the data of Figure 14 in a statistical form. For each of the three phases, 1, 2 and 3 the accelerometer (the top three bar charts) and gyroscope (the bottom three bar charts) signals are shown, with the mean and standard deviation of the relative contribution of each motion component represented (as a percentage). Thus, the bar height represents the mean for that axis relative to the other axes, and the margin bars represent the standard deviation.

For example, it can be seen that there are gradual transitions in $Y_{acc}$, $Z_{acc}$, and $X_{gyro}$. These characteristic features can be used in combination with the accurately measured bottle angle, to develop a regression model which maps these inputs to the correct infant inclination angle.

Figure 16 shows the results for the test data. The line 160 shows the actual bottle angle, and the grey regions indicate the time period during which the inclination angle was set for the different phases. Plot 162 is the predicted inclination angle of the infant obtained via linear regression. The prediction of the infant angle clearly adapts to the changes at each phase and thus departs from the bottle angle. If the inclination angle is determined solely based on the bottle angle, a significant underestimation would result.

The information obtained, of the tilt angle, the inclination angle, or both, may be used to derive feedback for the parent relating to the infant position. Optionally other feeding cues may be collected as well such as whether the feed was restless or stable, did the infant get cramp etc., which would provide the opportunity to identify optimal conditions for successful feeding.

The example above shows a sleeve, but any sensing arrangement positionally fixed with respect to the bottle may be used. It may be integrated into the bottle or a cap of the bottle.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A monitoring system for determining infant orientation information during bottle feeding, comprising:

   a sensor arrangement for obtaining bottle orientation information and movement information in respect of the feeding bottle during feeding; and a processor (22) adapted to identify from the sensor arrangement sensor signals infant orientation information in respect of the infant; and an output interface (20) for providing output information dependent on the infant orientation information.

2. A monitoring system as claimed in claim 1, wherein the sensor arrangement (18) comprises a three-axis motion sensor.

3. A monitoring system as claimed in claim 2, wherein the sensor arrangement (18) comprises a three-axis accelerometer and/or a three-axis gyroscope.

4. A monitoring system as claimed in any one of claims 1 to 3, wherein the output interface (20) comprises a wireless transmitter for sending the output information to a remote device (24) for presentation to a user.

5. A monitoring system as claimed in any one of claims 1 to 4, wherein the processor (22) is adapted to identify infant orientation information comprising an angle of tilt of a body axis of the infant about a vertical axis.

6. A monitoring system as claimed in any one of claims 1 to 5, wherein the processor (22) is adapted to translate the sensor arrangement signals to a reference coordinate system to compensate for rotation about a bottle longitudinal axis during a feed.

7. A monitoring system as claimed in claim 6, wherein the processor (22) is adapted to identify components of movement corresponding to longitudinal movement parallel to a body axis of the infant caused by jaw movement and thereby to determine the orientation of the body axis of the infant.

8. A monitoring system as claimed in claim 7, wherein the processor (22) is adapted to identify the components of movement by finding a minimum correlation between components of movement in orthogonal direction in a plane perpendicular to a longitudinal axis of the bottle.

9. A monitoring system as claimed in any one of claims 1 to 8, wherein the processor (22) is adapted to identify infant orientation information comprising an angle of tilt of a body axis of the infant about a horizontal axis.

10. A monitoring system as claimed in claim 9, wherein the processor (22) is adapted to identify an offset between a longitudinal axis of the bottle and an axis perpendicular to the body axis of the infant.

11. A monitoring system as claimed in claim 10, wherein the processor (22) is adapted to identify the offset by using a regression model which models the way bottle movements vary during feeding in dependence on the offset angle.

12. A monitoring system (12) as claimed in any one of claims 1 to 11, arranged as a sleeve for mounting around a feeding bottle.

13. A method for determining infant orientation information during bottle feeding, comprising:

   obtaining bottle orientation information and movement information in respect of the feeding bottle during feeding; and
   identifying from the bottle orientation information and movement information infant orientation information in respect of the infant; and
   providing output information dependent on the infant orientation information.

14. A method as claimed in claim 13, comprising:

   identifying infant orientation information comprising an angle of tilt of a body axis of the infant about a vertical axis; and/or
   identifying infant orientation information comprising an angle of tilt of a body axis of the infant about a horizontal axis.

15. A computer program comprising computer program code means which is adapted, when said program is run on a computer, to implement the method of claim 13 or 14.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

Europäisches
Patentamt

European
Patent Office

Office européen
des brevets

# EUROPEAN SEARCH REPORT

Application Number

EP 19 21 1379

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 110 339 067 A (CHENGDU BESTCARE TECH CO LTD) 18 October 2019 (2019-10-18) <br> * page 7, paragraph 49 - paragraph 50 * <br> * page 7, paragraph 38 * <br> * figures 1-2 * <br> * page 4, paragraph 9 * <br>----- | 1-4,6, 12,13,15 | INV. <br> A61B5/107 <br> A61B5/00 <br> A61J9/00 <br> G16H20/13 |
| A | WO 2017/056083 A1 (SHOHAM AFIK [IL]; SAGRON AVRAHAM [IL] ET AL.) 6 April 2017 (2017-04-06) <br> * page 10, line 17 - page 11, line 2 * <br> * page 13 - page 14; claims 1,8,9 * <br> * figures 1-5 * <br>----- | 1-15 | |
| A | US 2014/372045 A1 (KESKI-PUKKILA PANU MATTI [FI] ET AL) 18 December 2014 (2014-12-18) <br> * figure 11 * <br> * figure 3 * <br>----- | 1-15 | |
| A | EP 3 448 349 A1 (KONINKLIJKE PHILIPS NV [NL]) 6 March 2019 (2019-03-06) <br> * page 4, paragraph 22 - paragraph 25 * <br>----- | 1-15 | TECHNICAL FIELDS SEARCHED (IPC) <br><br> A61B <br> A61J <br> G16H |
| A | WO 2018/122173 A1 (KONINKLIJKE PHILIPS NV [NL]) 5 July 2018 (2018-07-05) <br> * figures 3-4 * <br>----- | 1-15 | |

The present search report has been drawn up for all claims

| Place of search | Date of completion of the search | Examiner |
|---|---|---|
| The Hague | 10 February 2020 | Hirsch, Arthur |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

EP 3 827 748 A1

**ANNEX TO THE EUROPEAN SEARCH REPORT
ON EUROPEAN PATENT APPLICATION NO.**

EP 19 21 1379

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2020

| Patent document cited in search report | | Publication date | Patent family member(s) | | Publication date |
|---|---|---|---|---|---|
| CN 110339067 | A | 18-10-2019 | NONE | | |
| WO 2017056083 | A1 | 06-04-2017 | EP | 3405164 A1 | 28-11-2018 |
| | | | US | 2019096224 A1 | 28-03-2019 |
| | | | WO | 2017056083 A1 | 06-04-2017 |
| US 2014372045 | A1 | 18-12-2014 | NONE | | |
| EP 3448349 | A1 | 06-03-2019 | BR | 112018071746 A2 | 19-02-2019 |
| | | | CN | 109069350 A | 21-12-2018 |
| | | | EP | 3448349 A1 | 06-03-2019 |
| | | | JP | 2019514527 A | 06-06-2019 |
| | | | US | 2019125630 A1 | 02-05-2019 |
| | | | WO | 2017186504 A1 | 02-11-2017 |
| WO 2018122173 | A1 | 05-07-2018 | NONE | | |

EPO FORM P0459

For more details about this annex : see Official Journal of the European Patent Office, No. 12/82